Europäisches Patentamt

European Patent Office

Office européen des brevets

(11) Publication number: **0 206 692**
**A2**

(12) **EUROPEAN PATENT APPLICATION**

(21) Application number: 86304571.2

(22) Date of filing: 13.06.86

(51) Int. Cl.⁴: **A61L 25/00** , **A61K 33/30**

(30) Priority: 14.06.85 GB 8515170

(43) Date of publication of application:
30.12.86 Bulletin 86/52

(84) Designated Contracting States:
AT BE CH DE FR GB IT LI LU NL SE

(71) Applicant: **Ed. Geistlich Söhne A.G. für Chemische Industrie**

**CH-6110 Wolhusen Lucerne(CH)**

(72) Inventor: **Geistlich, Peter**
**Haus Seldwyla Kehrsitenstrasse 19**
**CH-6362 Stansstad(CH)**

(74) Representative: **Holmes, Michael John et al**
**Frank B. Dehn & Co. Imperial House 15-19 Kingsway**
**London WC2B 6UZ(GB)**

(54) Topically administrable pharmaceutical compositions.

(57) A topically administrable wound-healing composition is decribed which comprises a physiologically acceptable zinc compound and one or more topical pharmaceutical carriers. The compositions are capable of sustained release of zinc ions and thereby promote granulation and tissue regeneration at the wound site.

EP 0 206 692 A2

## TOPICALLY ADMINISTRABLE PHARMACEUTICAL COMPOSITIONS

This invention relates to pharmaceutical compositions in forms suitable for topical administration to the human or animal body to promote the healing of wounds.

It is necessary with patients with extensive tissue damage, particularly wounds (including wounds resulting from injuries or surgical operations and burns), to protect the wound while tissue regrowth occurs. For this purpose various topically administered preparations, such as gels and ointments, have been used which maintain an acceptable environment at the tissue regeneration site, for example in terms of temperature and moisture. Such preparations may contain antibacterial agents to reduce or avoid infection of the wound site or growth promotors to enhance tissue regeneration.

It is an object of the present invention to provide a solid or semi-solid topically administrable composition capable of enhancing tissue regeneration at external wound sites.

We have found that tissue regeneration is surprisingly strongly enhanced by the inclusion of zinc in certain pharmaceutical preparations administered topically to external wound sites.

According to one aspect of the invention we therefore provide a pharmaceutical composition in solid or semi-solid form for topical application to external wounds to enhance tissue regeneration comprising a physiologically acceptable zinc compound and one or more topical pharmaceutical carriers, the composition being one capable of sustained release of zinc ions therefrom.

The compositions of the invention preferably also include the essential and semi-essential amino acids which as described in EP-A-47647 may also serve to promote wound healing.

The solid and semi-solid pharmaceutical compositions of the invention are advantageously in the form of smearable gels, creams, emulsions, balms or salves, powders, films or webs. The term "solid" in this context relates to gels having some resilience or dimensional stability, in contrast to "semi-solid" gels which are smearable and do not have such dimensional stability. The solid compositions particularly advantageously comprise as the carrier a solid (as distinct from semi-solid) water-permeable aqueous polymer gel film or web such as the acrylamide -agar films described in GB-A-2036042 of Max-Planck-Gesellschaft zur Forderung der Wissenschaften E.V. It is naturally desirable that such films or webs, which essentially act as bandages which are capable of releasing wound-healing medicaments, should be structurally stable, that is they should not be dissolved or resorbed by the fluids exuded by the wound. Furthermore, it is often desirable to apply such gel materials in powder form to provide a layer of aqueous gel over the wound and in this case also, it is preferred that the gel is not soluble or resorbable.

The carrier in the compositions of the invention serves essentially as a sustained release delivery system for the active zinc component whereby on application of the composition to a wound the zinc is delivered to the wound at a relatively constant rate over a prolonged period. This contrasts with prior art zinc ointments the zinc supply of which is quickly exhausted after application. With such compositions, daily application can mean that effective doses of zinc ions are supplied for only about 2 hours or so.

The compositions of the invention conveniently contain the zinc compound(s) in concentrations sufficient to give a zinc content of from 0.01 to 0.25, preferably 0.02 to 0.2 and especially preferably 0.03 to 0.10% by weight. The optimal concentration is 0.05% m/m. Above 0.25% Zn there is reduced epthelialisation although some cell proliferation is observed.

The compositions of the invention, particularly when in the form of aqueous gels or water-permeable aqueous polymer films or webs are preferably at substantially neutral pH to enhance their compatabiity with the wound site and will often be isotonic. The compositions may thus contain further ionic material. Suitable cations include magnesium, calcium, potassium and sodium; nevertheless it is preferred that sodium ion concentrations should be low and sodium ion free or substantially sodium ion free compositions are particularly preferred.

The counter-ions both for the zinc ions and for any further cations included (e.g. to adjust the ionic strength of the compositions) should clearly be physiologically acceptable anions which are compatible in the sense that they do not form insoluble compounds with zinc ions (or any other cations present); zinc ions may particularly suitably be incorporated into the compositions of the invention as the zinc salt of an acidic amino acid, e.g. zinc aspartate or zinc taurine, although zinc sulphate may also be a convenient source of zinc ions if the total zinc content is not to exceed about 0.25% by weight.

To adjust the pH of the composition to the desired level, physiologically acceptable, compatible acids or bases should be used, i.e. compounds which do not form insoluble salts or complexes with any of the other components. Particularly suitable acids for use in this respect are acetic, malic, hydrochloric and hydrobromic acids.

The compositions of the invention may optionally furthe contain growth promoting agents, such as for example fibronectin, a growth factor in the production of the protein "net" over a wound in the first stages of healing, or calmodulin, a calcium regulator, or chelating agents, such as for example EGTA (ethylene glycol tetraacetic acid).

The compositions of the invention conveniently include as pharmaceutical carriers or excipients compounds or materials which enable the compositions to be presented in topically administrable forms such as those recited above. Thus those carriers and excipients may be selected from those known in the art for the formulation of preparations suitable for treatment of external wounds.

In general, the compositions of the invention may be produced by the incorporation of zinc ions into solid water-permeable aqueous polymer gels or into semi-solid gels based on polysaccharides and derivatives thereof such as cellulose derivatives, e.g. carboxymethylcellulose (CMC), microcrystalline cellulose, alginate, agarose or tragacanth gum, or polyvinyl-pyrrolidone.

We have found that solid hydrogels, suitably in sheet form, provide extremely effective carriers for the zinc compounds; especially good results are obtained if the gels contain over 90%, preferably over 95% of water.

Hydrogel sheets may readily be formed from hot aqueous solutions of gel-forming natural or synthetic polymers, for example polysaccharides such as agar, carrageenin, alginates and pectins - (especially low methoxy pectins) and polypeptides and proteins such as gelatin. Alternatively, hydrogel sheets may be formed by polymerisation of watersoluble vinyl momomers in aqueous solution in the presence of a cross-linking agent such as a polyfunctional vinyl compound. Examples of such monomers include acrylic and methacrylic acid and their salts, amides and N-substituted amides, N-vinyl pyrrolidone and vinyl ethers of $C_{1-5}$ alkanols. Examples of cross-linking agents include ethylene glycol bis-acrylate, N,N'-methylene-bis-acrylamide, divinyl ether and diallyl maleate. It is also possible to produce hydrogel sheets by cross-linking water soluble polymers using cross-linking agents or ionising radiation followed by re-swelling with water; thus, for example, a film of polyvinyl alcohol may be cross-linked with ethylene diisocyanate while a sheet of high molecular weight polyethylene glycol may be cross-linked by irradiation with $Co^{60}$ gamma rays.

A still further possibility is to prepare cross-linked gelatin sheets by cross-linking aqueous gelatin with an aldehyde such as formaldehyde or glyoxal or by addition of a polyvalent metal salt such as aluminium sulphate.

Particularly good results may be expected using hydrogels which contain a gel-forming protein, polypeptide or polysaccharide (such as one of those mentioned above) the chains of which are interspersed with chains of a hydrophilic synthetic polymer (such as one of the vinyl polymers mentioned above). Particularly useful gel-forming proteins and polysaccharides include gelatin and agar. The synthetic polymer is preferably an acrylic polymer having hydrophilic groupings, polyacrylamide being especially useful. The cross-linking agent is preferably an acrylate compound having at least two double bonds, for example N,N'-methylene-bis-acrylamide.

Such gels are capable of retaining large amounts of water while having excellent mechanical strength and good surface properties such as smoothness and lack of tackiness.

The hydrogel may conveniently contain 2 to 10% of the protein, polypeptide or polysaccharide and hydrophilic polymer together with the hydrophylic synthetic polymer, preferably containing 1 to 5% of the protein, polypeptide or polysaccharide and 1 to 5% of the synthetic polymer.

The above solid water-permeable aqueous polymer gel compositions may advantageously comprise at least one gellable polysaccharide and/or protein or polypeptide and a cross-linked polymer of a hydrophilic acrylic acid or methacrylic acid derivative. The said hydrophilic-derivative is preferably an amide such as acrylamide. The gellable substance is preferably agar. The gel composition may be formed by polymerising the hydrophilic acrylic or methacrylic acid derivative together with a bifunctional acrylic cross-linking agent in admixture with a colloidal dispersion of the gellable substance.

The semi-solid pharmaceutical compositions of the invention are preferably in smearable gel-like form. By "semi-solid aqueous gel form" is meant a gel-like form which is spreadable or smearable, having the consistency of an ointment or cream. Such compositions may be in the form of colloidal dispersions of materials such as carboxymethylcellulose, agarose etc. which have little or none of the resilience or elasticity of a true gel but are particularly beneficial in releasing nutrients into the wound, as compared with solid or rigidly gelatinous nutrient formulations.

In general, where amino acids are present in the compositions of the invention their total concentration should be in the range 3-17%. However, the overall quantity of amino acid material partly depends on the nature of the individual amino acids. In many intravenous nutrient solutions, the total nitrogen supplied is predominantly in the form of simple amino acids such as glycine, alanine and glutamic acid. The body is capable of using these amino acids to synthesise a number of the non-essential amino acids present in human tissue. In general, however, this approach is less efficient than using a mixture of amino acids which approximates relatively closely to the proportions of the amino acids found in human tissues. It seems likely that the mechanisms available to the body for synthesis of other amino acids from glycine, alanine and glutamic acid may not be effective at the site of granulation of new tissue. For that reason, the amino acids are, in one embodiment of the invention, preferably substantially in the proportions found in human tissue and in that it is not intended that the quantities of glycine, alanine and glutamic acid should be high enough to provide significant levels of other amino acids, the overall concentration of amino acids in the gel according to the invention is preferably in the range 3 to 6% by weight.

It should be noted, however, that although the primary task of wound healing may be to regenerate skin, which consists largely of fibrous tissue, crude amino acid preparations derived by hydrolysis of collagen or elastin, which may be relatively inexpensive, do not contain all the essential amino acids and may contain peptide materials giving rise to reactions at the site of application.

When the composition of the invention is in the form of a hypertonic gel, due to relatively high concentrations of amino acids, the wound healing process may be promoted due to increased absorption of liquid secretion into the gel and increased movement of amino acids out of the gel. On the other hand, where the gel is substantially isotonic, it is more compatible with the granulating tissue.

The amino acids optionally used in the compositions of the invention are preferably present in the following quantities in g/kg composition.

| | | |
|---|---|---|
| L-Isoleucine | 1.3 - 5.7 | |
| L-Leucine | 2.1 - 8.8 | |
| L-Lysine HCl | 3.1 - 8.5 | Essential |
| L-Methionine | 1.8 - 7.5 | amino acids |
| L-Phenylalanine | 3.2 - 7.5 | |
| L-Threonine | 1.0 - 4.0 | |
| L-Tryptophan | 0.5 - 1.7 | |
| L-Valine | 1.6 - 6.7 | |
| | | |
| L-Arginine | 2.1 - 9.7 | Semi-essential |
| L-Histidine | 1.1 - 5.8 | amino acids |
| L-Alanine | 2.9 - 15.6 | |
| L-Glutamic acid | 1.9 - 21.6 | |
| Glycine | 2.0 - 25.0 | |
| L-Proline | 2.0 - 15.0 | |
| L-Ornithine - L-Aspartate | 1.0 - 8.5 | |
| L-Serine | 1.6 - 7.3 | |

It is highly preferred that all the essential and semi-essential amino acids are present in the compositions of the invention.

A preferred range of amino acid concentrations in g/kg composition is as follows:-

| L-Isoleucine | 1.8 – 5.4 |
|---|---|
| L-Leucine | 2.7 – 8.3 |
| L-Lysine HCl | 3.2 – 9.6 |
| L-Methionine | 2.2 – 6.8 |
| L-Phenylalanine | 3.2 – 9.8 |
| L-Threonine | 1.5 – 4.5 |
| L-Tryptophan | 0.6 – 2.0 |
| L-Valine | 2.1 – 6.3 |
| L-Arginine | 4.3 – 12.9 |
| L-Histidine | 1.0 – 3.2 |
| L-Alanine | 4.7 – 14.1 |
| L-Glutamic acid | 0.9 – 2.9 |
| Glycine | 2.0 – 6.2 |
| L-Proline | 5.6 – 16.9 |
| L-Ornithine – L-Aspartate | 0.9 – 2.9 |
| L-Serine | 1.0 – 3.5 |

It is particularly preferred that the compositions of the invention contain malic acid. This is directly beneficial in the regenerative process. The concentration of malic acid in the composition is preferably in the range 0.2 -0.6% by weight. The pH of the composition is preferably approximately neutral, i.e. in the range 6.5 -8, preferably 7.0 -7.5. It will be appreciated that in this pH range, many of the amino acids and the malic acid will be present partially in the form of salts.

The compositions of the invention conveniently include as pharmaceutical carriers or excipients compounds or materials which enable the compositions to be presented in topically administrable semi-solid aqueous gel forms. Carboxymethylcellulose is particularly suitably used as a gel-forming agent. However, other cellulose derivatives such as microcrystalline cellulose as well as polysaccharides such as alginate and agarose, tragacanth are also suitable as gel-forming agents.

It may be preferable for the gel-forming agent to be resorbable, particularly where there is a possibility of occlusion of the gel as the wound heals; resorbable gel-forming agents include dextrin, pectin and polyvinyl pyrrolidone. The gel may, if required, be made thicker and/or stiffer by addition of a relatively resilient gel-forming material such as a cross-linked fibrous protein, e.g. gelatin or collagen cross-linked with formaldehyde. Thus gelatin may be swelled with water to make an aqueous gel, contacted with formalin to effect cross-linking and subjected to washing to remove excess formaldehyde; the cross-linked gelatin may then be added to an amino acid solution, conveniently after removal of some or all of the water from the gel.

Where carboxymethyl cellulose is used as a gel-forming agent, this will normally be present as a salt. The sodium salt is readily available but where it is desired to reduce the concentration of sodium ions in the composition, another watersoluble salt may be desirable, for example the potassium salt or a salt with a physiologically acceptable organic base such as ethanolamine or triethanolamine or one of the basic amino acids, such as arginine or lysine, included in the amino acid component of the composition.

According to a further aspect of the invention we provide a method of treatment of the human or animal body to enhance tissue regeneration at an external wound site, the method comprising the topical administration at the external wound site of a solid or semi-solid pharmaceutical composition comprising a physiologically acceptable zinc compound and at least one topical pharmaceutical carrier capable of sustained release of zinc ions.

According to a yet further aspect of the invention we provide the use of a solid or semi-solid topically administrable pharmaceutical composition comprising a physiologically acceptable zinc compound and at least one topical pharmaceutical carrier capable of sustained release of zinc ions for the treatment of humans or animals to enhance tissue regeneration at external wound sites.

According to another aspect of the invention we provide the use of physiologically acceptable zinc compounds, particularly zinc salts of acidic amino acids, for the maufacture of a topical therapeutic agent capable of sustained release of zinc ions for the treatment of humans or animals to enhance tissue regeneration at external wound sites.

The compositions of the invention are intended for topical application to external wound sites, e.g. burns, accidental or surgical wounds and lesions, preferably at relatively long intervals, e.g. every 12-24 hours. When a fresh application is made, if the wound site is to be cleaned before the fresh application this should preferably be effected without exposing the regenerating tissue to temperatures significantly above or below body temperature and without abrasion of the site. Where the wound site is large and there is a significant danger of infection, it may be desirable to disinfect the site before application of the compositions of the invention. Suitable antibacterial substances for this purpose include Noxythioline, taurolidine, taurultam, iodine-povidone, chlorhexidine and antibiotics suitable for local administration such as amfomycin, bacitracin, neomycin, tyrothricin, polymyxin, as well as tetracyclines and amino glycosides.

The invention will now be further illustrated by the following Examples:

Example 1

3.5 g of acrylamide and 91 mg of methylenebisacrylamide are dissolved in 50 ml of distilled water. 2 g of agarose or agar-agar are dissolved in 50 ml of distilled water on the water bath at 100°C, allowed to cool to 60°C, and, after the addition of 60 $\mu$l of N,N,N',N'-tetramethylenediamine (TEMED) and 45 mg of peroxydisulfate, mixed with the acrylamide solution. The solution is poured immediately on to a glass plate measuring 12.5 $^x$ 26 cm and having a rim 2 mm high which has been preheated on a hot plate to 65°C. The latter is sealed with a glass cover such that no air bubbles are included. The cell is held for about 30 minutes at 56°C, until it is ascertained that the acrylamide has polymerized. After cooling, the plate is allowed to cure for at least 24 hours at 4°C. After removal, the gel is washed several times with one of the solutions set out in the following Table to remove unpolymerised material and to impregnate the gel with the aqueous composition under test. The gel sheets were subsequently granulated.

TABLE

| Sample | Zinc ion concentration (mMol) |
|--------|-------------------------------|
| 1 | 15.3 (zinc aspartate) |
| 2 | 30.6 (zinc aspartate) |
| 3 | 15.3 (zinc taurine) |
| 4 | 30.6 (zinc taurine) |
| 5 | 7.6 (zinc sulphate) |
| 6 | 15.3*[1] (zinc aspartate) |
| 7 | 15.3*[2] (zinc aspartate) |
| 8 | 15.3*[3] (zinc aspartate) |
| 9 | 15.3*[4] (zinc taurine) |

*1 -Solutions also containing 30.mmol Calcium (as calcium chloride), 2.34 mmol magnesium (as magnesium acetate) and 40.mmol potassium (as potassium chloride).

*2 -Solutions also containing 4 g/kg calcium chloride hexahydrate, 6 g/kg sodium chloride and 4 g/kg potassium chloride.

*3 -Solution components as for the gel of Example 2 but excluding the sodium carboxymethyl cellulose, glycerol and propylene glycol.

*4 -Solution components as for the gel of Example 4 but excluding the sodium carboxymethyl cellulose, glycerol and propylene glycol.

The compositions of the above samples were evaluated in tests on guinea pigs. In these tests wounds were created on the animals' backs and the granulated gel compositions and control compositions not containing the zinc compounds were applied topically to the wound sites. On subsequent removal of the composition, the granulation tissue that had formed on the wound site was removed and weighed.

It was found that a marked increase in granulation was observed on application of the above zinc containing compositions as compared to the controls.

EXAMPLE 2

1.0 kg of gel contains:

|  | | g | g |
|---|---|---|---|
| Sodium-carboxy-methylcellulose | | 13.000 | |
| Glycerol BP | | 10.000 | |
| Propyleneglycol | | 10.000 | |
| | | 33.000 | 33.000 |
| L-Isoleucine | | 3.586 | |
| L-Leucine | | 5.444 | |
| L-Lysine HCl | | 6.382* | |
| L-Methionine | | 4.506 | |
| L-Phenylalanine | | 6.459 | |
| L-Threonine | | 2.998 | |
| L-Tryptophan | | 1.315 | |
| L-Valine | | 4.206 | |
| L-Arginine | | 8.635 | |
| L-Histidine | | 2.060 | |
| L-Alanine | | 9.380 | |
| L-Glutamic Acid | | 1.876 | |
| Glycine | | 4.129 | |
| L-Proline | | 11.265 | |
| L-Ornithine – L-Aspartate | | 1.876 | |
| L-Serine | | 2.253 | |
| | | 76.370 | 109.370 |

|  | mmol** | | |
|---|---|---|---|
| Calcium chloride dihydrate | 30.0 | 4.411 | |
| Magnesium acetate anhydrate | 2.34 | 0.333 | |
| Potassium chloride | 40.0 | 2.982 | |
| | | 7.726 | 117.096 |
| Malic acid | | 4.318 | 121.414 |
| Zinc aspartate | 15.3 | | |
| Aqua dest. | | ad | 1000.000 |

\* (Base 5.016)

\*\* (Chloride ions Cl$^-$: 100.0 mmol
Acetate ions Ac$^-$: 4.68 mmol)

The glycerol and propylene glycol are added to the carboxymethylcellulose and mixed to produce a smooth dispersion. The amino acids, trace minerals and malic acid are dissolved in most of the water and the pH is adjusted to 7.2 with hydroxide. The above dispersion is added to the amino acid solution with continuous stirring, until free from undispersed solids and entrained air. The remaining water is then added to make up to the final volume. The resulting transparent, semi-solid gel is then autoclaved at 115°C -118°C for at least 30 minutes.

EXAMPLE 3

1.0 kg of gel contains:

|                                          | g        | g        |
|------------------------------------------|----------|----------|
| Potassium-carboxy-methylcellulose        | 13.000   |          |
| Glycerol BP                              | 10.000   |          |
| Propyleneglycol                          | 10.000   |          |
|                                          | 33.000   | 33.000   |
|                                          |          |          |
| L-Isoleucine                             | 3.586    |          |
| L-Leucine                                | 5.444    |          |
| L-Lysine HCl                             | 6.382*   |          |
| L-Methionine                             | 4.506    |          |
| L-Phenylalanine                          | 6.459    |          |
| L-Threonine                              | 2.998    |          |
| L-Tryptophan                             | 1.315    |          |
| L-Valine                                 | 4.206    |          |
| L-Arginine                               | 8.635    |          |
| L-Histidine                              | 2.060    |          |
| L-Alanine                                | 9.380    |          |
| L-Glutamic Acid                          | 1.876    |          |
| Glycine                                  | 4.129    |          |
| L-Proline                                | 11.265   |          |
| L-Ornithine – L-Aspartate                | 1.876    |          |
| L-Serine                                 | 2.253    |          |
|                                          | 76.370   | 109.370  |

|                                   | mmol**   |         |          |
|-----------------------------------|----------|---------|----------|
| Sodium chloride                   | 32.87    | 1.921   |          |
| Potassium chloride                | 28.04    | 2.090   |          |
| Magnesium acetate anhydrate       | 2.34     | 0.333   |          |
|                                   |          | 4.344   | 113.714  |
|                                   |          |         |          |
| Malic acid                        |          | 4.318   | 118.032  |
|                                   |          |         |          |
| Zinc aspartate                    | 30.6     |         |          |
|                                   |          |         |          |
| Aqua dest.                        |          | ad      | 1000.000 |

\*   (Base 5.016)

\*\*  (Chloride ions   Cl$^-$:  63.04 mmol
        Acetate ions   Ac$^-$:   4.68 mmol)

The gel is made up as in Example 2.

EXAMPLE 4

1.0 kg of gel contains:

|  | g | g |
|---|---|---|
| Sodium-carboxy-methylcellulose | 13.000 | |
| Glycerol BP | 10.000 | |
| Propyleneglycol | 10.000 | |
|  | 33.000 | 33.000 |
| | | |
| L-Isoleucine | 3.586 | |
| L-Leucine | 5.444 | |
| L-Lysine HCl | 6.382* | |
| L-Methionine | 4.506 | |
| L-Phenylalanine | 6.459 | |
| L-Threonine | 2.998 | |
| L-Tryptophan | 1.315 | |
| L-Valine | 4.206 | |
| L-Arginine | 8.635 | |
| L-Histidine | 2.060 | |
| L-Alanine | 9.380 | |
| L-Glutamic Acid | 1.876 | |
| Glycine | 4.129 | |
| L-Proline | 11.265 | |
| L-Ornithine - L-Aspartate | 1.876 | |
| L-Serine | 2.253 | |
|  | 76.370 | 109.370 |
| | | |
| Calcium chloride hexahydrate | 4.000 | |
| Sodium chloride | 6.000 | |
| Potassium chloride | 4.000 | |
|  | 14.000 | 123.370 |
| | | |
| Malic acid | 4.318 | 127.688 |
| Zinc taurine | 15.3 mmol | |
| Aqua dest. | ad | 1000.000 |

* (Base 5.016)

The gel is made up as in Example 2.

Example 5

Edible gelatin (125 g) is dispersed in 1% aqueous taurolidine (1250 ml) for about 10 minutes and subsequently warmed to 60°C with stirring. Aqueous formaldehyde (36%; 12 ml) is added to the liquid gel with stirring. The mixture is further stirred at 60°C for 10-15 minutes and then poured into clean pre-heated polyvinylchloride tubes (diameter 14 mm). The tubes are cooled overnight and cut into 15 cm lengths and cut open. The transparent rods so obtained are then washed in a 1% taurolidine solution for about 4 hours in order to remove excess formaldehyde. The formaldehyde is quantitatively detected by gas chromatography - (GC-WLD) and the washing is continued until no further free formaldehyde diffuses into the wash water. The detection limit for free formaldehyde by this method is 0.003%.

The rods are granulated in a Zyliss electric mincer, with sieve openings of 4.5 mm and dried in vacuo sufficiently to be milled to a fine powder.

5 mg portions of milled dried gel prepared as above are each added to 100 g of the aqueous formulation of each of the zinc-containing solutions in the Table in Example 1 and stirred until a smooth gel forms.

Example 6

20 g of agar-agar are suspended under agitation in 880 g of deionized water and heated to 95°C until complete dissolution. 1 l of a second aqueous solution containing 70 g of acrylamide and 1.84 g of N,N'-methylene-bis-acrylamide is prepared at ambient temperature and added to the first solution with thorough mixing. Under continued agitation, 2.2 g of N,N,N',N'-tetrakis-(2-hydroxypropyl)-ethylene diamine dissolved in 60 g of water and then 1.26 g of ammonium peroxydisulfate dissolved in 40 g of water are added.

The mixture has a temperature between 50°C and 55°C and begins to polymerize immediately. After 10 minutes the gel point is reached. The batch is allowed to cool down overnight during which time polymerization is completed.

The pale yellow, slightly turbid gel is cut into small 5 mm cubes and freed from soluble impurities by washing with pure flowing water for 24 hours. With this washing the gel swells to 135 % of its original weight.

100 parts by weight of washed gel are treated by soaking with 20 parts by weight of 10% aqueous ammonia solution for different lengths of time. After the treatment, excess liquid is drained off.

The batch is divided into two parts. One part is dried immediately in a circulating air blown oven at 45°C. The other part is treated at ambient temperature with 20 parts by weight of concentrated - (25%) ammonia solution per 100 parts of hydrogel for 24 hours. It is then dried in the same way. Both samples are ground to particle sizes smaller than 0.315 mm.

65 parts by weight of the dry hydrogel powder are thoroughly mixed in a ball mill with 1 part by weight of zinc aspartate. 6.6 g of the resultant mixture is transferred to a beaker and there swollen to a smooth gel paste (100 g) by the addition of distilled water (93.4 ml) under stirring with a spatula.

## Claims

1. A pharmaceutical composition in solid or semi-solid form for topical application to external wounds to enhance tissue regeneration comprising a physiologically acceptance zinc compound and one ot more topical pharmaceutical carriers, the composition being one capable of sustained release of zinc ions therefrom.

2. A pharmaceutical composition as claimed in claim 1 wherein the zinc concentration is from 0.01 to 0.25% by weight.

3. A pharmaceutical composition as claimed in claim 1 wherein the carrier is selected from water-permeable aqueous polymer gels and gels based on polysaccharide and derivatives thereof or polyvinylpyrrolidone.

4. A pharmeceutical composition as claimed in claim 1 in the form of a semi-solid gel which contains 3 to 17% by weight of amino acids.

5. A pharmaceutical composition as claimed in claim 4 wherein the amino acids comprise all the essential and semi-essential amino acids, present substantially in the proportions found in human tissue and at an overall concentration of from 3 to 6% by weight.

6. A pharmaceutical composition as claimed in claim 4 wherein sodium, potassum, calcium and/or magnesium ions are also present.

7. Use of physiologically acceptable zinc compounds for the manufacture of a topical therapeutic agent capable of sustained release of zinc ions for the treatment of humans or animals to enhance tissue regeneration at external wound sites.